# EUROPÄISCHE PATENTSCHRIFT

(11) **EP 0 996 472 B1**
(45) Veröffentlichungstag und Bekanntmachung des Hinweises auf die Patenterteilung: **09.02.2005**
(21) Anmeldenummer: 99920778.0
(22) Anmeldetag: 24.04.1999
(51) Int. Cl.: A61M 1/10, A61M 25/01

(54) **INTRAKARDIALE BLUTPUMPE**
INTRACARDIAC BLOOD PUMP
POMPE A SANG INTRACARDIAQUE

(30) Priorität: 13.05.1998 DE 19821307
(43) Veröffentlichungstag der Anmeldung: 03.05.2000
(73) Patentinhaber: Impella CardioSystems AG, 52074 Aachen (DE)
(72) Erfinder: SAMMLER, Rolf, 59348 Lüdinghausen (DE); SIESS, Thorsten, 52146 Würselen (DE); NIX, Christoph, 52076 Aachen (DE); EISEN, Max, 52074 Aachen (DE); HUTZENLAUB, Jens, Peter, 52064 Aachen (DE); MEYNS, Bart, University of Leuven, 3000 Leuven (BE)
(74) Vertreter: Selting, Günther, Dipl.-Ing.
(86) Internationale Anmeldenummer: PCT/EP1999/002790
(87) Internationale Veröffentlichungsnummer: WO 1999/058170

(56) Entgegenhaltungen:
- WO-A-94/09835
- DE-A- 19 622 335
- DE-U- 29 804 046
- US-A- 3 995 623
- NAKATANI T ET AL: "IN VITRO AND IN VIVO ASSESSMENT OF AN INTRAVENOUS AXIAL FLOW PUMP FOR RIGHT HEART ASSIST" ASAIO JOURNAL, Bd. 40, Nr. 3, 1. Juli 1994 (1994-07-01), Seiten 723-727, XP000498263 ISSN: 1058-2916

## Beschreibung

Die Erfindung betrifft eine intrakardiale Blutpumpe, und insbesondere eine Blutpumpe, die vollständig in das Herz über angrenzende Gefäße eingeführt werden kann, um die natürliche Pumpfunktion des Herzens zu unterstützen oder durch kontinuierlichen Pumpbetrieb zu ersetzen.

Eine Pumpvorrichtung zur Herzunterstützung ist beschrieben in WO94/09835 (Jarvik). Diese Pumpvorrichtung weist voneinander unabhängige Pumpen auf, die jeweils aus einem Pumpenteil und einem starr damit verbundenen Antriebsteil bestehen. Der Pumpenteil der einen Pumpe wird durch eine apekale Operationsöffnung hindurch derart in den linken Ventrikel eingeführt, daß er aus dem linken Ventrikel heraus in die Aorta fördert. Der andere Pumpenteil wird durch eine weitere, vorzugsweise apekale, Operationsöffnung hindurch in den rechten Ventrikel so eingeführt, daß er aus dem rechten Atrium in die Pulmonalarterie hineinfördert. Die Pumpen sind jeweils an ihrem Pumpenauslaß mit einer schlauchförmigen Auslaßkanüle verlängert, die durch die jeweilige Herzklappe hindurchgeführt werden kann.

Eine intrakardiale Blutpumpe, die von der Aorta durch die Aortenklappe hindurch in den linken Ventrikel eingeführt wird, ist bekannt aus WO97/37696 (Rau et al.). Hierbei ist der Pumpeneinlaß durch einen Schlauch verlängert, welcher durch die Aortenklappe hindurchgeht.

Aus WO97/37697 (Rau et al.) ist eine intravasale Blutpumpe bekannt, die durch ein Blutgefäß hindurchgeschoben werden kann. Auch diese Blutpumpe ist an ihrem Einlaßende durch einen Ansaugschlauch verlängert. Dieser Ansaugschlauch trägt einen aufblasbaren Ballon, welcher als Sperrvorrichtung dient, um einen seitlich an dem Ansaugschlauch vorbeiführenden Blutfluß zu verhindern.

Der Oberbegriff des Patentanspruchs 1 geht aus von einer intrakardialen Blutpumpe, wie sie in ASAIO Journal 1994, 723 - 727, beschrieben ist. Diese Blutpumpe weist ein Pumpenteil auf, an dessen Auslassende sich eine flexible Kanüle anschließt. Das Pumpensystem wird durch die femorale Vene in die Pulmonalarterie eingeführt, und ein Ballonkatheter führt sie dabei unter fluoroskopischer Führung zur Pulmonalarterie. An der Kanüle ist ein Ballon nicht vorgesehen.

In DE-A-196 22 335 ist ein Ballonkatheter beschrieben, der einen Pumpenteil aufweist, von dem ein rohrförmiger Ballonträger absteht. Auf dem Ballonträger sitzt ein Dilatationsballon, der sich über nahezu die gesamte Länge des Ballonträgers erstreckt und dazu dient, eine Stenose aufzuweiten, während von dem Pumpenteil Blut durch den Ballonträger perfundiert wird.

Schließlich ist aus US-Patent 4 753 221 (Kensey et al.) ein Pumpenkatheter bekannt, der eine im Herzen zu plazierende Pumpe aufweist. Diese Pumpe besteht aus einem Flügelrad und einem das Flügelrad umgebenden Pumpengehäuse. Das Pumpengehäuse kann im kollabierten Zustand im Herzen plaziert und anschließend durch Aufblasen eines mit dem Pumpengehäuse verbundenen Ballons aufgefaltet werden.

Ferner sind zu diagnostischen Zwecken kleinlumige Katheter zur Druck- und Volumenstromerfassung bekannt, die am distalen Katheterende einen aufblasbaren Ballon aufweisen. Dieser Ballon wird zur Plazierung der Katheterspitze in der Pulmonalarterie verwendet.

Bei intrakardialen Blutpumpen, die durch ein Blutgefäß hindurch in das Herz eingeführt werden, besteht die Schwierigkeit der korrekten Plazierung der Blutpumpe im Herzen. Insbesondere wenn die Blutpumpe durch die obere Hohlvene hindurch eingeführt wird, um Blut von dem rechten Atrium in die Pulmonalarterie zu pumpen, ist die korrekte Plazierung der Blutpumpe schwierig, weil die Pumpe eine Umlenkung um nahezu 180° durchführen muß, damit die Ansaugöffnung im rechten Atrium und die Auslaßöffnung in der Pulmonalarterie liegt. Hinzu kommt, daß in dem rechten Ventrikel zahlreiche Fäden und Sehnen vorhanden sind, die die Tricuspidalklappe festhalten und die Segel der Klappe gegen Durchschlagen sichern und die Formstabilität des Herzens gewährleisten. Zwischen solchen Sehnen und Fäden hindurch muß eine Blutpumpe, die durch eine Hohlvene hindurch eingeführt wird, geführt werden.

Der Erfindung liegt die Aufgabe zugrunde, eine intrakardiale Blutpumpe zu schaffen, die relativ leicht im Herzen plaziert werden kann.

Die Lösung dieser Aufgabe erfolgt erfindungsgemäß mit den im Patentanspruch 1 angegebenen Merkmalen.

Bei der erfindungsgemäßen Blutpumpe ist an den Pumpenauslaß ein flexibler Schlauch angesetzt, dessen distales Ende einen als Führungselement im Blutstrom wirkenden Ballon aufweist. Die Erfindung nutzt den Umstand aus, daß der natürliche Blutstrom im Herzen von der Hohlvene aus in das rechte Atrium durch den rechten Ventrikel hindurch in die Pulmonalarterie führt. Die Blutpumpe, die am distalen Ende des Schlauchs mit einem Ballon versehen ist, wird entlang eben dieses Weges eingeführt, wobei der in dem Blutstrom treibende Ballon automatisch seinen Weg von dem rechten Atrium in die Pulmonalarterie findet. Der Schlauchauslaß wird anschließend in der Pulmonalarterie stabilisiert, während der Pumpenteil sich im rechten Atrium befindet. Der Pumpenschlauch erfährt dabei eine Biegung von annähernd 180°. Auf diese Weise gelingt es, die Blutpumpe einschließlich des Pumpenschlauchs korrekt im Herzen zu plazieren, ohne daß ein abtastendes Anstoßen des Schlauchauslasses an den Herzkammerwänden erfolgen müßte, oder diagnostische Hilfsmittel (Röntgen oder Ultraschall) benötigt werden.

Intrakardial im Sinne der vorliegenden Erfindung umfaßt die Herzkammern (Ventrikel), die Vorhöfe und die angrenzenden Gefäßstümpfe.

Es ist zweckmäßig, wenn der Pumpenschlauch entsprechend seiner endgültigen Lage, die er im Herzen einnimmt, vorgebogen ist. Dies erfordert eine Biegung um mindestens etwa 150°. Zwar muß der Pumpenschlauch, um durch die Hohlvene hindurch eingeführt zu werden, gestreckt werden können, jedoch sollte er im entspannten Zustand eine U-Form oder V-Form annehmen. Besonders vorteilhaft ist es, wenn der Pumpenschlauch eine Biegesteifigkeit hat, die vom proximalen Ende zum distalen Ende hin abnimmt. Auf diese Weise kann das distale Ende als Wegfinder leicht bewegt werden, so daß der Ballon der natürlichen Blutströmung besser folgen kann.

Der Ballon muß nicht notwendigerweise direkt an dem Pumpenschlauch angebracht sein. Er kann auch an einem Katheter befestigt sein, der Bestandteil des Pumpenschlauchs ist und innen oder außen durch diesen hindurch verläuft. In jedem Fall muß zu dem Ballon ein Lumen verlaufen, durch das der Ballon aufgeblasen werden kann. Es kann zusätzlich ein zweites Lumen vorgesehen sein, in das ein Führungsdraht eingeführt wird, mit welchem das Vorschieben des Pumpenschlauches durch das Gefäßsystem erleichtert wird. Nach Entfernen des Führungsdrahtes kann dieses zweite Lumen als Druckmeßlumen verwendet werden.

Gemäß einer bevorzugten Ausführungsform ist vorgesehen, daß der Ballon als Ringballon den Pumpenschlauch umgibt. Dies hat den Vorteil, daß das distale Schlauchende durch den Ballon einen vergrößerten Außendurchmesser aufweist, und dadurch von der Pulmonalklappe festgehalten wird. Auf diese Weise trägt der Ballon zur Verankerung des Schlauchendes an der Pulmonalklappe bei, während sich der Schlauchauslaß in der Pulmonalarterie befindet. Dadurch wird verhindert, daß der Schlauchauslaß aus der Pulmonalarterie herausgleitet. Ein weiterer Vorteil besteht darin, daß der Ringballon ein abgerundetes stumpfes Ende des Pumpenschlauches bildet, so daß mit dem Pumpenschlauch keine Verletzung der Gefäß- und Herzwände oder anderer Herzteile hervorgerufen werden können. Schließlich wird auch verhindert, daß das Schlauchende sich am Klappenrand oder an Sehnen im Herzen verhakt.

Da der Pumpenteil Blut in den Pumpenschlauch hineinpumpt, wird der Pumpenschlauch selbsttätig offengehalten. Als Pumpenschlauch kann daher ein kollabierbarer Schlauch benutzt werden, beispielsweise ein Folienschlauch, dessen Wand nicht formstabil ist. Durch diesen Schlauch hindurch kann ein Katheter zu dem am distalen Ende angeordneten Ballon verlaufen.

Bei einer intrakardialen Blutpumpe mit einem flexiblen Pumpenschlauch besteht die Gefahr, daß das aus dem Schlauchauslaß ausströmende Blut am Schlauch einen Rückstoß verursacht, welcher eine retrograde Verschiebung des Schlauchs zur Folge haben kann. Auf diese Weise kann es geschehen, daß der Schlauch aus der Pulmonalklappe herausgleitet. Der Erfindung liegt daher die weitere Aufgabe zugrunde, eine intrakardiale Blutpumpe zu schaffen, bei der Verschiebungen des Schlauchs infolge von hydraulischen Reaktionskräften vermieden werden.

Die Lösung dieser Aufgabe erfolgt erfindungsgemäß mit den Merkmalen des Patentanspruchs 13. Hiernach ist am distalen Ende des Schlauchs ein Zugelement befestigt, welches von dem Pumpenstrom angeströmt wird. Bei diesem Zugelement kann es sich um ein Segel handeln oder um einen Ballon. Der aus dem Schlauchende austretende Blutstrom stößt gegen das Zugelement, wodurch eine der Rückzugskraft des Schlauch entgegenwirkende, nach vorne gerichtete Kraft entsteht. Auf diese Weise dient das Zugelement der Stabilisierung der Positionierung des Pumpenschlauchs.

Im folgenden werden unter Bezugnahme auf die Zeichnungen Ausführungsbeispiele der Erfindung näher erläutert.

Es zeigen:
- Fig. 1: eine Querschnittsdarstellung des Herzens mit darin angeordneter Blutpumpe,
- Fig. 2: eine Darstellung der Blutpumpe von Fig. 1,
- Fig. 3: einen Schnitt durch die Einzelheit III von Fig. 2,
- Fig. 4: ein zweites Ausführungsbeispiel der Blutpumpe,
- Fig. 5: ein drittes Ausführungsbeispiel und
- Fig. 6: in vergrößertem Maßstab eine Darstellung des Katheters aus dem Ausführungsbeispiel von Fig. 5.

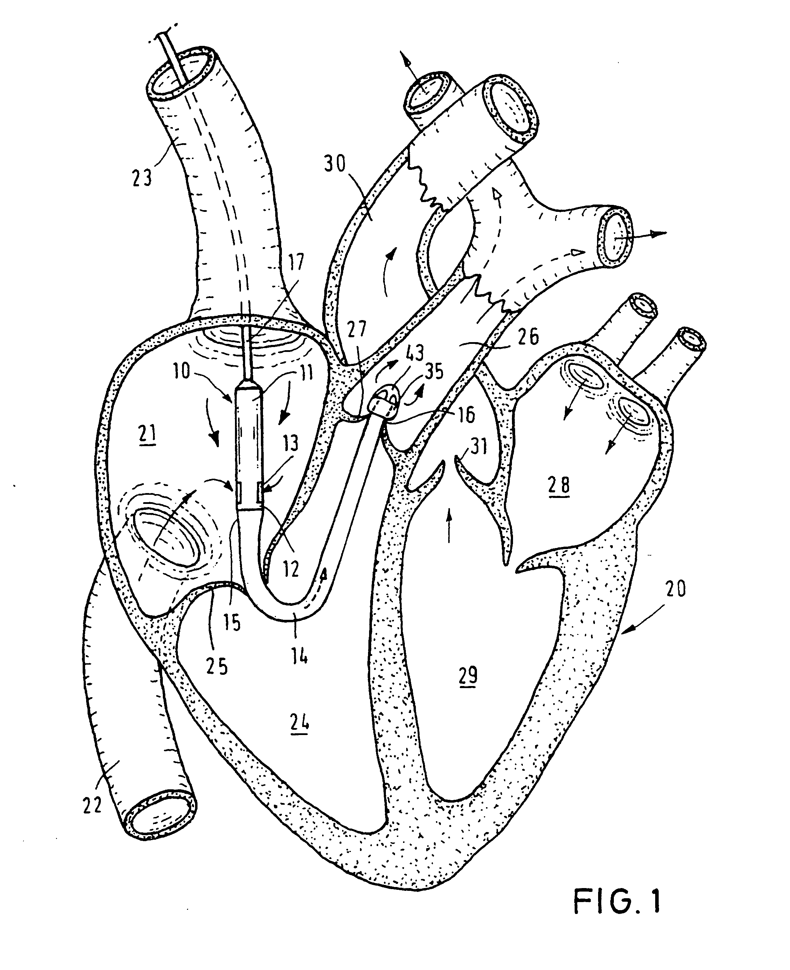

Gemäß Fig. 1 ist die Blutpumpe 10 eine intravasale Blutpumpe, also eine Blutpumpe, die durch das Blutgefäßsystem eines Patienten geschoben werden kann, um bis in das Herz hinein vorzudringen. Der Außendurchmesser einer solchen Blutpumpe ist an keiner Stelle größer als 8 mmm. Die Pumpe 10 weist einen Antriebsteil 11 und einen damit starr verbundenen Pumpenteil 12 auf. Die Pumpe entspricht beispielsweise derjenigen von WO97/37696, so daß ihr interner Aufbau hier nicht näher erläutert wird. Im Übergangsbereich zwischen Antriebsteil 11 und Pumpenteil 12 befinden sich Ansaugöffnungen 13, durch die Blut radial von außen her angesaugt wird. Der Pumpenteil 12 enthält ein (nicht dargestelltes) Flügelrad, das das Blut in axialer Richtung antreibt. An den Auslaß des Pumpenteils 12 ist ein Pumpenschlauch 14 angeschlossen. Dieser weist ein mit dem Pumpenauslaß verbundenes proximales Ende 15 und ein den Schlauchauslaß bildenes distales Ende 16 auf.

Das proximale Ende der Pumpe 10 ist mit einem Katheter 17 verbunden, welcher auch die elektrischen Versorgungsleitungen für den Antriebsteil 11 enthält. Ferner weist der Katheter 17 ein Lumen auf, das an eine Druckluftquelle angeschlossen werden kann.

Der Pumpenschlauch 14 und der Katheter 17 sind flexibel. Die rigide Länge der Pumpe 10 beträgt nicht mehr als 35 mm, damit die Pumpe durch Krümmungen des Blutgefäßsystems hindurchgeht. Die Länge des Pumpenschlauchs 14 ist mindestens doppelt so groß wie die rigide Länge der Pumpe. Der Pumpenschlauch hat eine Länge von etwa 12 cm und einen Außendurchmesser von etwa 8 mm. Seine Wandstärke beträgt 0,05 mm bis 0,2 mm. Der Krümmungsradius der Schlauchbiegung beträgt 40 mm bis 60 mm.

In Fig. 1 ist ein Querschnitt durch das Herz 20 dargestellt. In das rechte Atrium 21 münden die untere Hohlvene 22 und die obere Hohlvene 23. Zwischen dem rechten Atrium 21 und dem rechten Ventrikel 24 befindet sich die Tricuspidalklappe 25. Zwischen dem rechten Ventrikel 24 und der Pulmonalarterie 26 befindet sich die Pulmonalklappe 27. Von der Pulmonalarterie 26 fließt das Blut zur Lunge und von dort zurück in das linke Atrium 28 und zum linken Ventrikel 29. Zwischen dem linken Ventrikel 29 und der Aorta 30 befindet sich die Aortenklappe 31.

Die Pumpe 10 wird als Rechtsherzpumpe so verlegt, daß sie von dem rechten Atrium 21 in die Pulmonalarterie 26 hinein fördert. Hierzu ist sie bei dem dargestellten Ausführungsbeispiel durch die obere Hohlvene 23 hindurch verlegt. Es wäre auch möglich, die Verlegung durch die untere Hohlvene 22 hindurch vorzunehmen. Während des Einführens der Pumpe 10 liegt der Pumpenschlauch 14 der Pumpe voran, d.h. er liegt in Strömungsrichtung des die Pumpe umgebenden Blutes stromab von der Pumpe 10.

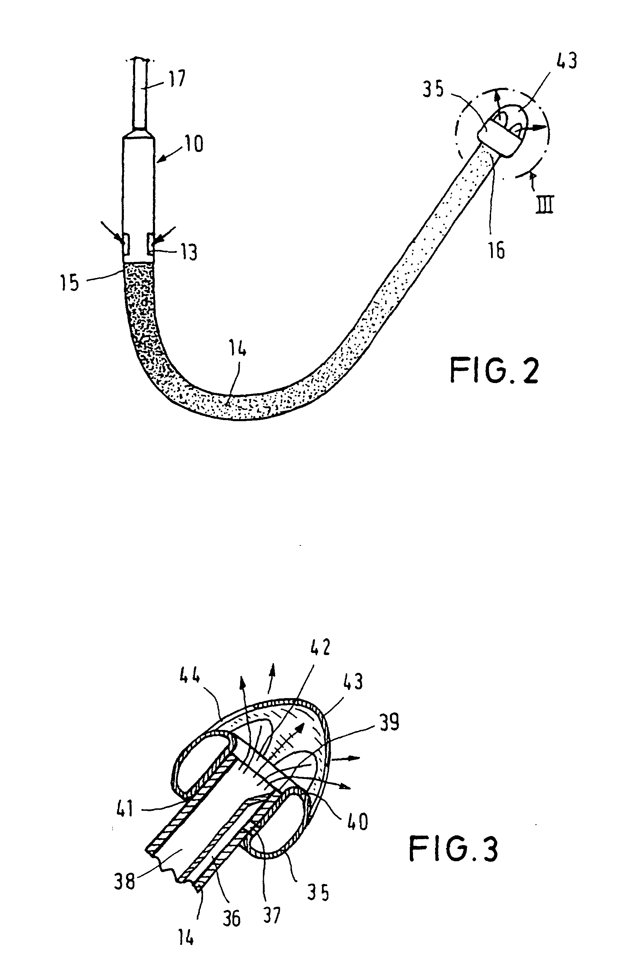

Am distalen Ende 16 des Pumpenschlauchs 14 befindet sich ein Ballon 35, der hier gemäß Fig. 2 und Fig. 3 als Ringballon ausgebildet ist. Der mit Gas (z.B. Luft, Helium, CO₂) oder einer Flüssigkeit aufgeblähte Ballon 35 hat einen größeren Außendurchmesser als der Pumpenschlauch 14. Folglich bildet der Ballon 35 ein Führungselement, an dem der natürliche Blutstrom angreift, um dieses Führungselement mitzuziehen. Dadurch wird der Ballon 35 auf dem natürlichen Blutweg zunächst in den rechten Ventrikel 24 und danach in die Pulmonalarterie 26 gespült.

Der Ballon 35 wird dadurch aufgebläht, daß in einem Drucklumen 36 des Pumpenschlauchs 14 ein Druck erzeugt wird. Das Drucklumen 36 steht über Öffnungen 37 mit dem Inneren des ringförmigen Ballons 35 in Verbindung. Im Pumpenschlauch 14 fließt das Blut durch das Blutlumen 38, dessen Querschnitt wesentlich größer ist als derjenige des Drucklumens 36. Das Drucklumen 36 ist an ein entsprechendes Drucklumen des proximalen Katheters 17 angeschlossen, so daß sein Druck extrakorporal gesteuert werden kann.

Der Pumpenschlauch 14 ist in Fig. 2 im entspannten Zustand dargestellt. Der Pumpenschlauch ist U-förmig oder V-förmig vorgebogen, d.h. er hat ein Formgedächtnis, so daß er im Herzen gemäß Fig. 1 keinem wesentlichen Zwang ausgesetzt ist und auch seinerseits keinen wesentlichen Zwang auf das Herz ausübt. In Fig. 2 gibt die Schwärzung des gepunkteten Bereichs die Steifigkeit des Pumpenschlauchs 14 an. Am proximalen Ende 15 ist diese Steifigkeit sehr groß und sie nimmt dann stetig zum distalen Ende 16 hin ab. Dies bedeutet, daß das distale Ende 16, an dem sich der Ballon 35 befindet, durch den Ballon frei bewegt und geführt werden kann.

Wie aus Fig. 3 hervorgeht, steht der Ballon 35 am distalen Ende über das Ende 39 des Pumpenschlauchs 14 hinaus vor, so daß der Ballon 35 ein abgerundetes Schlauchende 40 bildet, welches nicht die Gefahr des Verhakens an Hindernissen bietet. An dem proximalen Ende des Ballons 35 bildet dieser eine ringförmige Hinterschneidung 41, in die gemäß Fig. 1 die Spitzen der Pulmonalklappe 27 eindringen können, so daß die Pulmonalklappe ein Zurückweichen des Schlauchendes verhindert.

Wie in den Fign. 2 und 3 dargestellt ist, spannt sich über dem Schlauchauslaß 42 ein Zugelement 43 in Form eines kuppelförmigen Segels. Dieses Zugelement 43 besteht aus einer dünnen flexiblen Haut, die mit Stegen 44 an dem Ballon 35 befestigt ist. Das aus dem Schlauchauslaß 42 ausströmende Blut strömt gegen das Zugelement 43 und übt somit auf den Pumpenschlauch 14 einen Zug aus, der der Rückstoßwirkung entgegengesetzt ist. Dadurch wird verhindert, daß das distale Ende 16 des Pumpenschlauchs sich infolge des hydraulischen Rückstoßes verschiebt und evtl. aus der Pulmonalklappe 27 herausgleitet.

Der Pumpenschlauch 14 besteht vorzugsweise aus Polyurethan, das sich als besonders geeignet erwiesen hat.

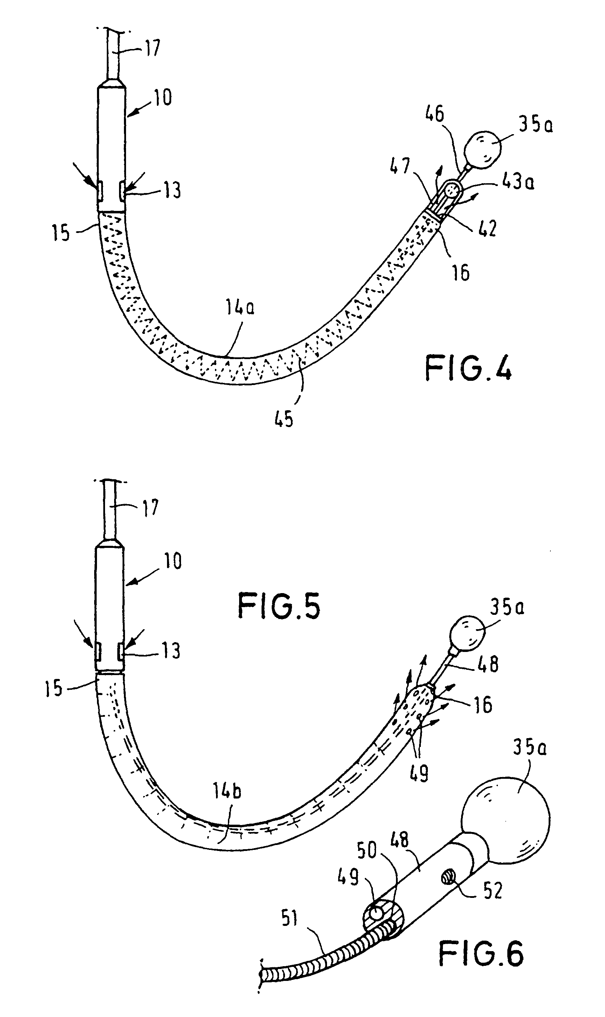

Bei dem Ausführungsbeispiel von Fig. 4 ist an die Pumpe 10 ein Pumpenschlauch 14a angeschlossen, der eine elastische Stützstruktur 45 in Form einer aus Carbon oder Metall bestehenden Schraubenwendel enthält. Diese Stützstruktur 45 hält den Pumpenschlauch offen und bewirkt die gewünschte Biegesteifigkeit, die auch über die Schlauchlänge variieren kann. Durch den Pumpenschlauch 45 hindurch verläuft ein Katheter 46, der am distalen Ende mit einem Ballon 35a versehen ist. Dieser Ballon bildet das Führungselement für den Pumpenschlauch 14a. Der Katheter 46 enthält ein Drucklumen. Sein Außendurchmesser ist wesentlich kleiner als der Durchmesser des Pumpenschlauchs 14a, so daß im Pumpenschlauch genügend Querschnitt für den Blutfluß zur Verfügung steht. Der Katheter 46 ist mit dem Katheter 17 verbunden.

Als Zugelement 43a ist eine Kugel im Abstand hinter dem Schlauchauslaß 42 angeordnet und am Katheter 46 befestigt. Die gegen das Zugelement 43a drückende Blutströmung verhindert ein Zurückweichen des distalen Schlauchendes 16 zur Pumpe 10. Der Pumpenschlauch 14a ist mit einer Haltevorrichtung 47 an dem Zugelement 43a verankert.

Bei dem Ausführungsbeispiel der Fign. 5 und 6 ist der Pumpenschlauch 14b ein kollabierbarer Pumpenschlauch, der aus dünnem Folienmaterial besteht, das keine eigene Formstabilität aufweist. Durch den Pumpenschlauch 14b verläuft ein Katheter 48, der an seinem distalen Ende einen Ballon 35a trägt. Dieser Katheter 48 ist mit dem Katheter 17 verbunden. Das distale Ende 16 des Pumpenschlauchs 14b ist mit dem Katheter 48 verbunden und der Pumpenschlauch 14b weist Austrittsöffnungen 49 für den Blutaustritt auf. Beim Einführen der Pumpe nach Fig. 5 ist die Pumpe nicht in Betrieb, so daß der Pumpenschlauch 14b kollabiert ist. Mittels des Ballons 35a wird der weiche und flexible Katheter 48 im Herzen verlegt, wobei er den Pumpenschlauch 14b positioniert. Wird anschließend die Pumpe in Betrieb gesetzt, so wird der Pumpenschlauch 14b aufgeweitet.

Zur Erleichterung der Plazierung kann der Katheter 36,36,48 gemäß Fig. 6 außer dem zum Ballon 35a führenden Drucklumen 49 ein weiteres Lumen 50 enthalten, das zur Aufnahme eines Führungsdrahtes 51 und nach Entfernen des Führungsdrahtes zur externen Druckmessung bestimmt ist. Dieser Führungsdraht 51, der auch durch den Katheter 17 und die Pumpe 10 hindurchgeht, ermöglicht es dem Operateur, die Verlegung des Pumpenschlauchs steuernd zu beeinflussen. Danach wird der Führungsdraht 51 herausgezogen.

Gemäß Fig. 6 kann an dem Katheter 48 eine Öffnung 52 vorgesehen sein, die mit dem Lumen 50 verbunden ist und die durch den Führungsdraht 51 versperrt wird. Nachdem der Führungsdraht 51 aus dem Lumen 50 herausgezogen ist, dringt durch die Öffnung 52 Blut in das Lumen 50 ein. Das Lumen 50 kann mit einem Blutdruckmesser verbunden werden, um somit den Blutdruck in der Pulmonalarterie während des Pumpens zu messen und ggf. beeinflussen zu können.

Erfolgt die Plazierung des Pumpenschlauches ohne Führungsdraht, so kann mit Hilfe der meßbaren Druckverläufe im weiteren Lumen 50 der Druck an der Stelle 52 gemessen werden. Aus den Druckverläufen kann so die genaue Position der distalen Pumpenschlauchspitze ermittelt werden.

Der Durchmesser des Ballons darf nicht so groß sein, daß er den Durchfluß durch die Pulmonalarterie 26 wesentlich behindert oder die Pulmonalarterie sogar absperrt. In der Regel darf der Durchmesser nicht größer sein als 30 mm. Ferner sollte der Ballon eine hohe Elastizität haben, im Unterschied zu einem Dilatationsballon. Als Ballonmaterial eignen sich wegen der Elastizitätseigenschaften Silikon, Latex, und vorzugsweise Polyurethan.

Der Ballon kann, während er als Führungselement für den Katheterschlauch im Blutstrom floatet, mit hohem Druck stark aufgebläht sein und anschließend durch Verringerung des Druckes verkleinert werden, um als Zugelement zu wirken, das von der Pumpenströmung angeströmt wird.

## Patentansprüche

1. Intrakardiale Blutpumpe, mit einem radial ansaugenden und axial fördernden Pumpenteil (11), der mit einem Katheter (17) verbunden ist, und einem mit dem Pumpenauslaß verbundenen flexiblen Pumpenschlauch (14), welcher ein an den Pumpenauslaß angrenzendes proximales Ende (15) und ein den Schlauchauslaß bildendes distales Ende (16) aufweist,
**dadurch gekennzeichnet,**
**daß** der Pumpenschlauch (14) nahe seinem distalen Ende und entfernt von dem Pumpenteil (11) einen von dem Blutstrom mitnehmbaren, als Führungselement wirkenden Ballon (35) aufweist.

2. Blutpumpe nach Anspruch 1, **dadurch gekennzeichnet, daß** der Pumpenschlauch um mehr als 100°, vorzugsweise um etwa 150°, vorgebogen ist.

3. Blutpumpe nach Anspruch 1 oder 2, **dadurch gekennzeichnet, daß** der Pumpenschlauch (14) eine Biegesteifigkeit hat, die vom proximalen Ende (15) zum distalen Ende (16) hin abnimmt.

4. Blutpumpe nach einem der Ansprüche 1-3, **dadurch gekennzeichnet, daß** der Pumpenschlauch (14) innen oder außen einen Katheter (46;48) aufweist, der das distale Ende (16) des Pumpenschlauchs überragt und jenseits des distalen Endes den Ballon (35a) trägt.

5. Blutpumpe nach einem der Ansprüche 1-3, **dadurch gekennzeichnet, dass** der Ballon (35) als Ringballon den Pumpenschlauch (14) umgibt.

6. Blutpumpe nach Anspruch 5, **dadurch gekennzeichnet, dass** der Ringballon eine abgerundete Schlauchspitze (40) bildet.

7. Blutpumpe nach einem der Ansprüche 1-6, **dadurch gekennzeichnet, dass** der Pumpenschlauch (14b) ein kollabierbarer Schlauch ist.

8. Blutpumpe nach einem der Ansprüche 1-7, **dadurch gekennzeichnet, dass** der Pumpenschlauch (14a) eine elastische Stützstruktur (45) enthält.

9. Blutpumpe nach einem der Ansprüche 1-8, **dadurch gekennzeichnet, dass** der Pumpenschlauch (14b) einen Katheter (48) aufweist, dass ein in den Katheter (48) einführbarer Führungsdraht (51) vorgesehen ist.

10. Blutpumpe nach einem der Ansprüche 1-9, **dadurch gekennzeichnet, dass** der Katheter (48) ein Lumen (50) für den Führungsdraht (51) und ein Drucklumen (49) aufweist.

11. Blutpumpe nach einem der Ansprüche 1-10, **dadurch gekennzeichnet, dass** der Katheter (48) ein als Druckmeßlumen benutzbares Lumen (50) aufweist.

12. Blutpumpe nach einem der Ansprüche 1-11, **dadurch gekennzeichnet, daß** der Durchmesser des Ballons im aufgeblähten Zustand nicht größer ist als 30 mm.

13. Blutpumpe nach einem der Ansprüche 1 - 12, **dadurch gekennzeichnet, daß** an dem distalen Ende (16) des Pumpenschlauchs (14) ein von dem Pumpenstrom angeströmtes Zugelement (43) befestigt ist.

## Claims

1. Intracardiac blood pump comprising a radially intaking and axially delivering pump portion (11) connected with a catheter (17), and a pump hose (14) connected with the pump outlet, which pump hose (14) has a proximal end (15) adjacent to the pump outlet and a distal end (16) forming the hose outlet,
**characterized in that**
the pump hose (14) comprises near its distal end and remote from the pump portion (11) a balloon (35) capable of being entrained by the blood flow and acting as guide element.

2. Blood pump according to claim 1, **characterized in that** the pump hose is pre-bent by more than 100°, preferably by approximately 150°.

3. Blood pump according to claim 1 or 2, **characterized in that** the pump hose (14) has a flexural rigidity which decreases from the proximal end (15) towards the distal end (16).

4. Blood pump according to one of claims 1-3, **characterized in that** the pump hose (14) comprises on its inside or its outside a catheter (46; 48) which projects beyond the distal end (16) of the pump hose and comprises the balloon (35a) beyond the distal end.

5. Blood pump according to one of claims 1-3, **characterized in that** the balloon (35) as annular balloon surrounds the pump hose (14).

6. Blood pump according to claim 5, **characterized in that** the annular balloon forms a rounded hose tip (40).

7. Blood pump according to one of claims 1-6, **characterized in that** the pump hose (14b) is a collapsible hose.

8. Blood pump according to one of claims 1-7, **characterized in that** the pump hose (14a) contains an elastic supporting structure (45).

9. Blood pump according to one of claims 1-8, **characterized in that** the pump hose (14b) comprises a catheter (48) and that a guide wire (51) capable of being inserted into the catheter (48) is provided.

10. Blood pump according to one of claims 1-9, **characterized in that** the catheter (48) comprises a lumen (50) for the guide wire (51) and a pressure lumen (49).

11. Blood pump according to one of claims 1-10, **characterized in that** the catheter (48) comprises a lumen (50) which can be used as pressure measuring lumen.

12. Blood pump according to one of claims 1-11, **characterized in that** the diameter of the balloon in the inflated condition does not exceed 30 mm.

13. Blood pump according to one of claims 1-12, **characterized in that** at the distal end (16) of the pump hose (14) a traction element (43) is fixed to which the pumped flow is directed.

## Revendications

1. Pompe à sang intracardiaque comportant une partie de pompe (11) aspirant radialement et refoulant axialement, reliée à un cathéter (17), et un tuyau de pompe (14) flexible relié à la sortie de pompe, le tuyau de pompe présentant une extrémité (15) proximale adjacente à la sortie de pompe, et une extrémité (16) distale formant la sortie du tuyau, **caractérisée en ce que** le tuyau de pompe (14) présente, à proximité de son extrémité distale et à distance de la partie de pompe (11), un ballon (35) pouvant être entraîné par le flux sanguin, agissant comme un élément de guidage.

2. Pompe à sang selon la revendication 1, **caractérisée en ce que** le tuyau de pompe est coudé à plus de 100°, de préférence à environ 150°.

3. Pompe à sang selon la revendication 1 ou 2, **caractérisée en ce que** le tuyau de pompe (14) possède une résistance à la flexion décroissante de l'extrémité proximale (15) à l'extrémité distale (16).

4. Pompe à sang selon l'une quelconque des revendications 1 à 3, **caractérisée en ce que** le tuyau de pompe (14) présente un cathéter (46, 48) à l'intérieur ou à l'extérieur, qui dépasse de l'extrémité distale (16) du tuyau de pompe et porte le ballon (35) de l'autre côté de l'extrémité distale.

5. Pompe à sang selon l'une quelconque des revendications 1 à 3, **caractérisée en ce que** le ballon (35) entoure le tuyau de pompe comme un ballon circulaire.

6. Pompe à sang selon la revendication 5, **caractérisée en ce que** le ballon circulaire forme une pointe de tuyau (40) arrondie.

7. Pompe à sang selon l'une quelconque des revendications 1 à 6, **caractérisée en ce que** le tuyau de pompe (14b) est un tuyau pliable ou écrasable.

8. Pompe à sang selon l'une quelconque des revendications 1 à 7, **caractérisée en ce que** le tuyau de pompe (14a) comporte une structure de support (45) élastique.

9. Pompe à sang selon l'une quelconque des revendications 1 à 8, **caractérisée en ce que** le tuyau de pompe (14b) présente un cathéter (48), et **en ce qu'**un fil de guidage (51) pouvant être guidé dans le cathéter (48) est prévu.

10. Pompe à sang selon l'une quelconque des revendications 1 à 9, **caractérisée en ce que** le cathéter (48) présente une lumière (50) pour le fil de guidage (51) et une lumière artérielle (49).

11. Pompe à sang selon l'une quelconque des revendications 1 à 10, **caractérisée en ce que** le cathéter (48) présente une lumière (50) utilisable comme lumière de mesure de la pression artérielle.

12. Pompe à sang selon l'une quelconque des revendications 1 à 11, **caractérisée en ce que** le diamètre du ballon, à l'état gonflé, ne dépasse pas 30 mm.

13. Pompe à sang selon l'une quelconque des revendications 1 à 12, **caractérisée en ce qu'**un élément de traction (43) gonflé par le courant de pompage est fixé à l'extrémité distale (16) du tuyau de pompe (14).
